# EUROPEAN PATENT SPECIFICATION

(11) **EP 4 334 768 B1**
(45) Date of publication and mention of the grant of the patent: **22.07.2026**
(21) Application number: 22727853.8
(22) Date of filing: 04.05.2022
(51) Int. Cl.: G02B 21/00, G01N 15/10, G02B 21/26, G02B 21/34, A61B 10/00

(54) **A MOBILE SLIDE IMAGING KIT AND USE THEREOF**
MOBILES OBJEKTTRÄGERBILDGEBUNGSKIT UND VERWENDUNG DAVON
KIT D'IMAGERIE À LAME MOBILE ET SON UTILISATION

(30) Priority: 04.05.2021 GB 202106375
(43) Date of publication of application: 13.03.2024
(73) Proprietor: Micron Agritech Limited, Dublin (IE)
(72) Inventor: IZQUIERDO HIJAZI, Daniel, Dublin 6 (IE); LOPEZ ESCOBAR, Jose, Dublin 7 (IE); SMITH, Sean, Dublin 12 (IE); RICHARDS, Brian, Dublin 11 (IE); DAS, Vineeth, Dublin 3 (IE); MCELLIGOTT, Tara, Dublin 15 (IE)
(74) Representative: Tomkins & Co
(86) International application number: PCT/EP2022/062068
(87) International publication number: WO 2022/233985

(56) References cited:
- EP-A1- 3 401 665
- WO-A1-2020/059554
- WO-A1-2020/120640
- WO-A1-2021/219898
- CN-A- 110 987 768
- KR-B1- 101 926 059
- US-A- 5 569 607
- US-A1- 2015 054 935
- XU DANDAN ET AL: "Automatic smartphone-based microfluidic biosensor system at the point of care", BIOSENSORS AND BIOELECTRONICS, vol. 110, 1 July 2018 (2018-07-01), Amsterdam , NL, pages 78 - 88, XP055953345, ISSN: 0956-5663, DOI: 10.1016/j.bios.2018.03.018
- ALDO RODA ET AL: "Smartphone-based biosensors: A critical review and perspectives", TRAC TRENDS IN ANALYTICAL CHEMISTRY, vol. 79, 1 May 2016 (2016-05-01), AMSTERDAM, NL, pages 317 - 325, XP055617846, ISSN: 0165-9936, DOI: 10.1016/j.trac.2015.10.019
- BALLWEBER L R ET AL: "American Association of Veterinary Parasitologists' review of veterinary fecal flotation methods and factors influencing their accuracy and use-Is there really one best te", VETERINARY PARASITOLOGY, vol. 204, no. 1, 30 July 2014 (2014-07-30), pages 73 - 80, XP029035167, ISSN: 0304-4017, DOI: 10.1016/J.VETPAR.2014.05.009

## Description

### Field of the Invention

The present invention relates to a slide imaging kit and in particular to a kit for imaging a faecal egg counting slide. The invention also relates to methods of using a kit to image a slide.

### Background to the Invention

The faecal egg count (FEC) is a primary diagnostic tool of veterinary parasitology. Increasing levels of anthelmintic resistance in commonly occurring parasites of farm animals has necessitated the routine use of FECs as a monitoring tool for evaluating treatment efficacy and overall parasite egg shedding level.

Numerous techniques exist for determining FECs, including the McMaster (Gordon and Whitlock, 1939), Stoll (Stoll, 1923), Wisconsin (Cox and Todd, 1962) and Mini-FLOTAC (Cringoli et al., 2017) methods. All of these are underpinned by a single principle, which is the separation of parasite ova from the bulk of the faecal detritus by flotation in a dense liquid medium, and subsequent microscopic examination and manual counting of the ova. The McMaster method is currently recommended for use in veterinary practice. The McMaster slide comprises two square faecal flotation chambers; each configured to hold approximately 0.15 ml. At the magnification required to view and count eggs (about 10x), only a part of the chamber can be viewed at any time. This problem is addressed by providing a printed grid that demarcates each chamber into six grid areas each of which has an elongated rectangular shape. Counting of eggs in each chamber is performed on a grid-by-grid basis, necessitating continuous slow movement of the stage of the microscope relative to the microscope lens to examine each grid area, and to move from grid area to grid area. This has been found to time intensive, require significant training, and lead to significant operator error. This is particularly an issue when the slide is being examined remotely (i.e. not in a lab, but on a farm for example). A further problem with the McMaster slide is that it is made from two glass plates that abut on assembly, an upper flat glass plate including the printed grid, and a lower glass plate that is contoured and requires machining for manufacture. This makes the slide expensive to manufacture.

EU legislation (Directive EU 2019/6) will lead to an increase in FEC testing to combat the resistance issue through TST (targeted selective treatment). In addition, studies have found that through TST (based on an indicator like an FEC) the same health & yield output can be achieved in animals with just 24% of the medication input. This supports the need for a testing system that it is easy to use and amenable to being used on farms by farmers. Such a system is described in WO2020120640. The system is configured for use in the field, and includes a sample preparation kit, and a slide reader for taking images of a faecal egg slide, where in one embodiment a mobile phone is coupled to the slide reader to take images of the faecal egg slide and relay the images to a remote location for analysis, and analysis data is relayed back to the phone. The system is very useful for providing near-real time analysis of the health of an animal using a kit which is easy to use even in a farm setting. Providing fast information on the health of an animal allows fast decisions to be made in the field with regard to which animals should be administered anti-parasite drugs, and also allows the health of individual animals to be tracked. Figure 9A and 9B of WO2020120640 describe a specific slide reader for use with a McMaster slide having a slide reader that is configured to receive a slide and couple with a mobile phone to allow the camera of the phone image the square grid flotation chamber of the slide through the lens of the reader. A problem with this device is that the area of the flotation chamber on the slide is large and it is difficult to scan the whole area, especially when the analysis is performed in a remote location such as a farm. In addition, there is no disclosure of how the phone is coupled to the reader to ensure correct alignment between the phone camera and the lens of the slide body, and no disclosure of how the slide can be moved on the viewing platform in both the X and Y direction. Moreover, there is no way to keep the slide focused on the same plane and no system for mounting the lens disclosed. In addition, there is no disclosed way to allow for different types of tests to be done with the same system. Further, EP 3 401 665 A1 discloses a system for imaging biological samples using a mobile device. The system comprises a cassette having a closed elongated sample-receiving channel and an optical assembly including a lens. The cassette is moved relative to the optical system by a motor so that the entire length of the channel can be scanned. A mobile device such as a smartphone is coupled to the optical assembly for capturing images of the sample.

It is an object of the invention to overcome at least one of the above referenced problems.

### Summary of the Invention

The objective is met by the provision of a mobile kit comprising a slide reader that is configured to receive a slide that is provided with a liquid sample chamber that is generally linear and elongated, and generally has a length that is at least 10 or 20 times greater than the width. This allows the full length of the chamber to be imaged by moving the slide in a single linear (or circular) motion relative to the slide reader, allowing an imaging device that is coupled to the slide reader record a series of images or a video along most of all of the length of the slide. The slide reader has a lens for imaging the chamber of the slide and that may be configured such that the width of the chamber corresponds to at least 60% or 80% of the field of view of the lens of the slide. The mobile kit is dimensioned to be easily portable, and easily usable, allowing use in the field (for example on a farm) using a mobile phone as the imaging device which is coupled to the slide reader. The invention also describes an attachment module which is designed to allow the imaging device (e.g. a phone) to be easily and accurately aligned with the slide body so that the imaging device can image the liquid sample chamber of the slide through the lens of the slide reader. The attachment module is a planar body that can be easily manipulated by the user, and has an aperture that is aligned the imaging device (e.g. the camera of a phone) easily, where alignment can be easily checked by flipping the attachment module over and checking if the camera is aligned with the aperture. A face of the attachment may include fixing means such as a dry adhesive for fixing the phone in the aligned position. Once aligned, the attachment module is then attached to the slide reader, and both the attachment module and slide reader may be modified with corresponding formations (e.g. corresponding magnets or chamfering) dimensioned or configured to facilitate correct alignment obviating the need for it to be aligned visually. Another aspect of the invention is the provision of a track for the slide in the slide reader, and magnets on a base of the slide and on a base of the track which serve to maintain the slide in contact with base of the track as it is moved along the track. This prevents vertical movement of the slide during imaging ensuring that the quality of the images or video is not compromised. The kit of the invention may be used to image various types of microscopic microorganisms such as faecal eggs of parasitic works or larvae. With faecal eggs, the liquid sample chamber is a flotation chamber configured to allow any faecal eggs in the sample float to the top, and the lens of the slide body and imaging device are configured to have a focal point at or adjacent to a top of the chamber. The provision of a visual indicium disposed on a top of the slide at an inlet end of the chamber may be used to focus the imaging device on the top of the chamber. The provision of first and second visual indicia disposed at each end of the chamber may be employed to start and stop a drive module configured to move the slide along the track. Without the indicia, automated starting and stopping would require an expensive wired or wireless communication between the reader and slide like a Bluetooth connection. Thus, the slide has been specifically built for automated analysis which cannot be done with the McMaster slide.

In a first aspect, the invention provides a mobile kit for imaging microscopic organisms in a liquid sample on a slide. The organisms may be parasites, for example eggs, larvae or worms. In one embodiment, the slide is a faecal egg imaging slide. The kit comprises a slide reader containing a lens and configured to receive a slide and image a liquid sample chamber of the slide through the lens using a mobile imaging device that is coupled to the slide reader. The liquid sample chamber of the slide is a linear elongated liquid sample chamber.

The slide reader comprises a track configured to receive the slide and guide movement of the slide along the track allowing the length of the elongated liquid sample chamber to be imaged through the lens. The movement is linear.

The slide reader comprises a drive module configured to move the slide relative to the track. Slide reader firmware may be updated via a wireless or wired connection. It will be appreciated that this connection may be via the mobile imaging device or an alternative computing device.

In embodiments falling under the scope of the claims, the kit comprises a slide having a linear elongated liquid sample chamber.

In embodiments falling under the scope of the claims, the kit comprises a substantially planar attachment module having an upper surface, a lower surface, and an aperture extending between the surfaces. The upper surface is configured for detachably attaching to a mobile imaging device. The lower surface is configured to detachably attach to the slide reader such that a lens of the mobile imaging device is aligned with the lens of the slide reader through the aperture.

In any embodiment, the lower surface of the attachment module and upper surface of the slide reader comprise magnets configured to guide the alignment of the attachment module and slide body.

In any embodiment, an upper surface of the slide body and lower surface of the attachment module are shaped (e.g. comprise chamfered portions) to guide the alignment of the attachment module and slide body.

In any embodiment, the upper surface of the attachment module comprises a layer of dry adhesive such as synthetic setae.

In any embodiment, the slide comprises formations (e.g. a series of teeth) disposed along the slide, generally a side of the slide. In any embodiment, the drive mechanism of the slide body comprises a pinion configured to engage the formations (e.g. teeth) and rotate to drive movement of the slide, typically in a linear motion along the track.

In any embodiment, the drive mechanism is configured to move the slide relative to the track at a speed of 0.5 to 10 mm/s, 0.5 to 5 mm/s, 0.5 to 2 mm/s or 0.5 to 1.5 mm/s.

In any embodiment, the lens of the slide reader has an objective lens and an eyepiece lens. In any embodiment the objective lens has a magnification of 5-20x, 5-15x, 10-20x, or 10-16x. In any embodiment the eyepiece lens has a magnification of 15-25x or 20-22xx

In any embodiment, a lower surface of the slide comprises one or magnets, and in which a base of the track comprises one or more magnetic strips.

In any embodiment, the slide comprises a lens module, a base module, and a slide receiving module containing the track and drive mechanism connected between the lens module and base module.

In any embodiment, the lens module is detachably attached to the slide receiving module.

In any embodiment, the track comprises an illumination section disposed directly under the lens, and the slide body comprises a light disposed under the illumination section to direct light through the illumination section on to a base of the slide during use.

In any embodiment, the track is disposed horizontally across the slide body and is configured to receive the slide on one side of the slide housing and dispense the slide on an opposite side of the slide housing.

In any embodiment, the lens of the slide body is configured to provide a field of view when imaged by the mobile imaging device, in which the width of the linear elongated liquid sample chamber comprises at least 60%, 70%, 80%, 90% or 95% of the field of view.

In any embodiment, the linear elongated liquid sample chamber has a length at least 20 times greater than its width.

In any embodiment, the linear elongated sample chamber has a length of 50 to 100 mm.

In any embodiment, the linear elongated sample chamber has a width of 0.5 to 5 mm,

In any embodiment, the linear elongated sample chamber has a height of 1 to 5 mm.

In any embodiment, the linear elongated sample chamber has a volume of about 0.5 to about 10 ml, typically about 1 to about 5 ml, typically about 2 to about 4 ml, and preferably about 3 ml.

In any embodiment, the slide body has an upper face and a first indicium disposed on the upper face at an inlet end of the slide body in a position that axially aligned with a longitudinal axis of the elongated flotation chamber. The indicium is generally a symbol such as a letter or number that allows a lens to focus. In use the imaging device is trained to recognise the first indicium and modify its focal length to bring the indicium into focus. This is especially useful for imaging of faecal eggs as the indicium is disposed on a top of the slide adjacent to a plane of the top of the liquid sample chamber where faecal eggs will be located.

In any embodiment, the slide comprises a second indicium disposed on the upper face at an outlet end of the slide body in a position that axially aligned with a longitudinal axis of the elongated flotation chamber. The second indicium may be the same as the first indicium or different. The imaging device is trained to recognise the indicia and initiate the drive mechanism of the slide reader when the fist indicium is recognised and stop the drive mechanism when the second indicium is recognised.

In any embodiment, the indicium has a maximum dimension of less than 5 mm, 4 mm, 3 mm, or 2mm. Maximum dimension of less that X mm means that the indicium can fit within a circle having a diameter of X mm.

In any embodiment, a ratio of the track length to slide length is 1.0 : 0.5 to 0.5 to 1.0, preferably about 0.85 : 1.0 to 1.0 to 0.85.

In any embodiment, the upper face of the slide body comprises a visible line disposed on each longitudinal side of the elongated flotation chamber and co- parallel and optionally co-extensive with the flotation chamber. These lines may be used by the imaging device or a processor to determine the size of objects such as faecal eggs in the chamber. Generally, each visible line is spaced apart from the elongated flotation chamber by a distance of less than 1 mm, for example 0.2 mm or 0.3 mm.

In any embodiment, an upper surface of the slide is transparent and a lower surface of the slide is semi-transparent to diffuse light passing through the slide from beneath the slide. This allows viewing of the sample chamber through the transparent cover layer and also provides for diffusion of light emitted from a source under the slide ensuring that the length of the sample chamber is illuminated.

In any embodiment, the mobile imaging device is a mobile phone with a camera.

In any embodiment, the slide is a faecal egg enumeration slide.

In any embodiment, the slide comprises:
a base layer having an upper face and a lower face, in which the upper face comprises a peripheral shoulder section and a recessed central section; and
a cover layer dimensioned to nest on top of the recessed central section,
wherein the recessed central section comprises an elongated channel with an open top, and wherein the elongated liquid sample chamber is defined by the elongated channel and cover layer when nested in the recessed central section.

**In** any embodiment, the liquid sample inlet of the linear elongated liquid sample is disposed on a top surface of the slide.

In any embodiment, the slide comprises a sample inlet chamber disposed in fluid communication with the liquid sample inlet and an inlet end of the linear elongated liquid sample chamber.

In any embodiment, the cover layer is transparent and the base layer is translucent and light-diffusing.

In any embodiment, the mobile kit comprises software for a mobile communications device having a camera, in which the software is configured to cause the mobile communications device to:
recognise using the camera the first indicium disposed on the upper face of the slide adjacent an inlet end of the linear elongated liquid sample chamber; and
focus the lens of the camera on the first indicium.

In any embodiment, the software is configured to cause the mobile communications device to:
recognise using the camera the second indicium disposed on the upper face of the slide body adjacent an outlet end of the elongated flotation chamber;
determine if the second indicium is in focus; and
alert a user if the second indicium is not in focus.

In any embodiment, the mobile kit comprises software for a mobile communications device having a camera, in which the software is configured to:
recognise using the camera the first indicium disposed on the upper face of the slide body adjacent an inlet end of the elongated flotation chamber;
initiate recording of images or video by the camera along the length of the elongated flotation chamber in response to recognition of the first indicium;
recognise using the camera the second indicium disposed on the upper face of the slide body adjacent an outlet end of the elongated flotation chamber; and
stop recording of images or video by the camera in response to recognition of the second indicium.

In any embodiment, the upper face of the slide comprises a visual line disposed on each side and co-parallel and optionally co-extensive with the linear elongated liquid sample chamber.

In any embodiment, the kit comprises software configured to compare the distance between the visual lines with the size of a microscopic object in the linear elongated liquid sample chamber and provide an estimate of the size of the microscopic object based on the comparison.

In any embodiment, the kit comprises software configured to recognise the two lines and orient the images or video recorded by the imaging device such that the lines are disposed horizontally across the image when the recorded image or video is displayed on a graphical display.

In any embodiment, the kit comprises machine learning software.

In any embodiment, the kit comprises firmware that may be updated through wired or wireless communication means.

In any embodiment, the kit comprises software configured to perform one or more functions using the data (e.g. images or video) received from the imaging device, for example:
identify eggs and/or their types or bubbles;
calculate the size of each of the objects detected and categorise each object detected into its correct egg type (optionally discards any mistakes of the machine learning software);
tracks each object and counts how many times it appears to produce an egg count (quantitative analysis); and/or
generate a report and send the report to the user.

In another aspect, the invention provides a method of imaging microscopic organisms in a liquid sample according to claim 14.

The slide may be loaded and placed into the slide reader either before or after the attachment module is attached to the imaging device and slide reader.

In any embodiment, the method comprises a step of recording images or a video of the full length of the linear elongated sample chamber by the mobile imaging device.

In any embodiment, the method is a method of enumerating faecal eggs in a liquid sample, in which the linear elongated liquid sample chamber is faecal egg flotation chamber, in which the method includes a step of enumerating faecal eggs along the length of the elongated flotation chamber from the images or video.

In any embodiment, the mobile imaging device is a mobile phone with a camera.

In any embodiment, the method comprises a step of transmitting by the mobile phone of the images or video to a remote location.

In any embodiment, the slide comprises a first indicium disposed on the upper face of the slide adjacent an inlet end of the linear elongated liquid sample chamber, in which the method comprises the steps of:
recognising by the mobile imaging device the first indicium; and
focusing a lens of the mobile imaging device on the first indicium.

In any embodiment, the slide comprises a second indicium disposed on the upper face of the slide adjacent an outlet end of the linear elongated liquid sample chamber, in which the method comprises the steps of:
recognising by the mobile imaging device the second indicium;
determining if the second indicium is in focus; and
alerting a user if the second indicium is not in focus.

In any embodiment, the slide comprises a first indicium disposed on the upper face of the slide adjacent an inlet end of the linear elongated liquid sample chamber and a second indicium disposed on the upper face of the slide adjacent an outlet end of the linear elongated liquid sample chamber, in which the method comprises the steps of:
recognising by the mobile imaging device the first indicium disposed on the upper face of the slide adjacent an inlet end of the linear elongated liquid sample chamber;
initiating recording of images or video by the mobile imaging device along the length of the linear elongated liquid sample chamber in response to the recognition by the mobile imaging device of the first indicium;
recognising by the mobile imaging device the second indicium disposed on the upper face of the slide body adjacent an outlet end of the linear elongated liquid sample chamber; and
stopping recording of images or video by the mobile imaging device in response to recognition by the imaging device of the second indicium.

In any embodiment, the upper face of the slide body comprises a line disposed on each side and co-parallel with the linear elongated liquid sample chamber, in which the method comprises the steps of:
comparing by the imaging device the distance between the lines with the size of a microscopic object in the linear elongated liquid sample chamber; and
providing an estimate of the size of the microscopic object based on the comparison.

In any embodiment, the upper face of the slide body comprises a line disposed on each side and co-parallel with the linear elongated liquid sample chamber, in which the method comprises the steps of:
recognising by the mobile imaging device the two lines in the recorded images or video; and
orienting the images or video such that the lines are disposed horizontally across the image when the recorded image or video is displayed on a graphical display.

In any embodiment, the drive module moves the slide along the track at a speed of 0.5 to 10 mm/s, 0.5 to 5 mm/s, 0.5 to 3 mm/s or 0.1 to 1.5 mm/s mm/s.

In any embodiment, the method comprises interrogating the data from the imaging device (e.g. images or video) with software configured to:
identify eggs and/or their types or bubble;
calculate the size of each of the objects detected and categorise each object detected into its correct egg type (optionally discards any mistakes of the machine learning software);
tracks each object and counts how many times it appears to produce an egg count (quantitative analysis); and/or
generate a report and send the report to the user.

In any embodiment, the software is machine learning software.

Other aspects and preferred embodiments of the invention are defined and described in the other claims set out below.

### Brief Description of the Figures

**FIG. 1** is a perspective view of a faecal egg counting slide according to the invention.
**FIG. 2** is a perspective view of the faecal egg counting slide of Figure 1 with the cover layer removed.
**FIG. 3** is a plan view from above the slide.
**FIG. 4** is a sectional view taken along the line B-B of Figure 3.
**FIG. 5** is a sectional view taken along the line A-A of Figure 3.
**FIG. 6** is a rear perspective exploded perspective view of the faecal egg counting slide of Figure 2.
**FIG. 7** is a front perspective exploded perspective view of the faecal egg counting slide of Figure 2.
**FIGS. 8A and 8B** are a perspective views of a kit according to the invention comprising a slide reader and attachment module.
**FIG. 9** is an exploded perspective view of the kit of Figure 8.
**FIG. 10** is a top plan view of the kit of Figure 8 showing the attachment module.
**FIG. 11** is a sectional view taken along the lines A-A of Figure 10.
**FIG. 12** is a sectional view of the top of the slide reader showing the lens module and attachment module prior to engagement.
**FIG. 13** is a perspective view showing the lens module and attachment module engaged.
**FIG. 14** is a perspective view of the slide receiving module and a slide tilted away from the track to show the magnetic strips on the base of the track.
**FIG. 15** is a top plan view of the slide receiving module and base module of Figure 14.
**FIG. 16** is a top plan view of the kit of Figure 8 showing the phone attachment module and a slide partly inserted into the slide receiving module.
**FIG. 17** is a perspective view of the kit of Figure 8 showing a mobile phone attached to the phone attachment module and a slide partly inserted into the slide receiving module.

### Detailed Description of the Invention

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or openended and does not exclude additional, unrecited integers or method/process steps.

### Exemplification

The invention will now be described with reference to specific Examples. These are merely exemplary and for illustrative purposes only: they are not intended to be limiting in any way to the scope of the monopoly claimed.

These examples constitute the best mode currently contemplated for practicing the invention.

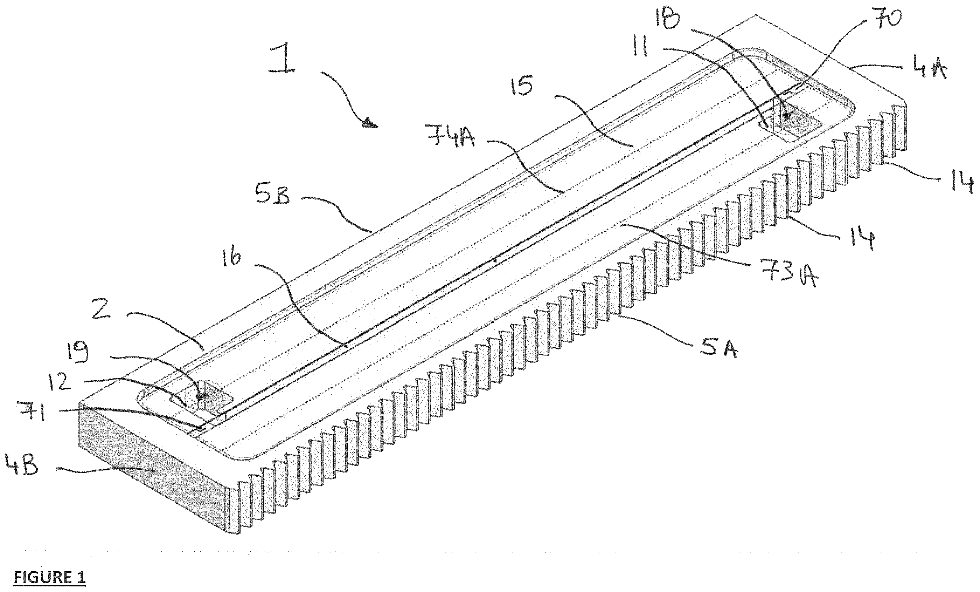

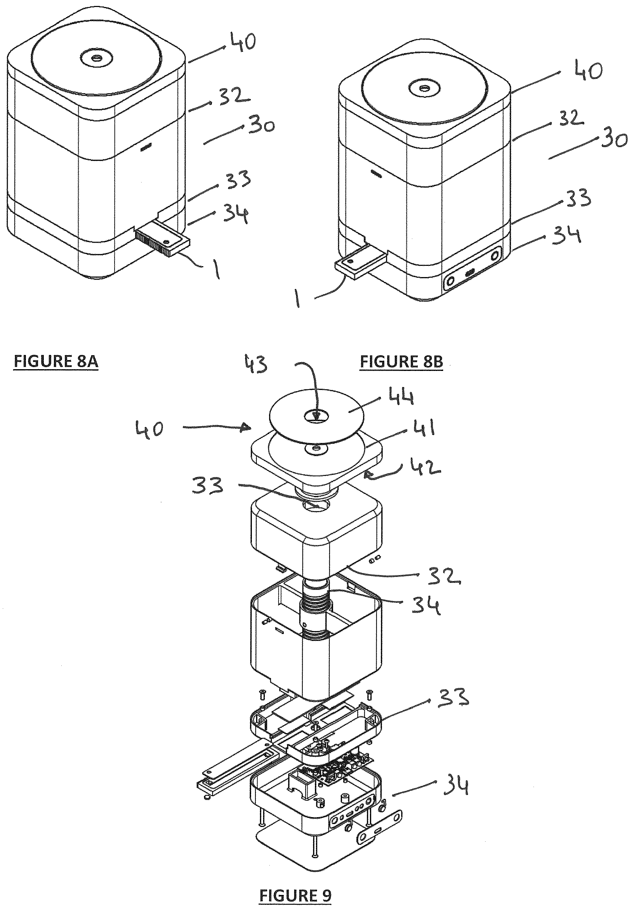

Referring to the drawings, there is illustrated a mobile kit according to the invention for use in imaging a faecal egg slide for the purpose of enumerating faecal eggs on the slide. The mobile kit is suitable for use in a remote location such as on a farm. Figures 1 to 7 illustrate a slide indicated by the reference numeral 1 and figures 8 to 18 illustrate a slide reader 30 and attachment module 40 for an imaging device, in this case a mobile phone with a camera.

Referring to Figures 1 to 7, there is illustrated a slide for use with, or forming part of, a mobile kit of the invention, indicated generally by the reference numeral 1 having an upper surface 2, lower surface 3, ends 4A, 4B, and sides 5A, 5B. The slide is generally rectangular and has approximate dimension of 90 mm (length), 20 mm (width), and 5 mm (height). The upper surface 2 has a rectangular recess 7 defining a peripheral shoulder 8, and an elongated channel 10 is formed in the recess. A sample inlet chamber 11 is provided at an inlet end of the channel and an outlet chamber 12 is provided at an outlet end of the channel. The purpose of the inlet channel is to allow liquid sample to be added into the channel without bubble formation. Referring specifically to Figure 1, the slide includes a cover plate 15 dimensioned to nest in the recess 7 and, together with the elongated channel 10, define a linear elongated liquid sample chamber 16. The cover plate 15 includes an inlet aperture 18 and outlet aperture 19 positioned to align with the inlet chamber 11 and outlet chamber 12 when the cover plate 15 is nested in the recess.

In this embodiment, the linear elongated liquid sample chamber 16 has a length of 84 mm, a width of 1 mm, and a depth of 3.6 mm. The chamber 16 has a rectangular profile along the full length of the chamber and has a volume of about 3 ml.

Referring to Figure 1, the cover plate 15 has a first indicium 70 at an inlet end of the elongated chamber 16 and a second indicium 71 at an outlet end of the chamber 16. Both the first and second indicia are the letter "L" having a height of any 1 mm. The indicia provide a means by which the camera and imaging device can focus on the chamber, and start and finish recording images or video. They are also used to determine if the chamber is in focus after video or images have been recorded along the length of the chamber. The cover plate also has two lines 73A and 74A printed on the surface which extend the full length of the cover plate parallel to the chamber and spaced on each side of the chamber by about 4 mm. These lines are used to provide a distance reference used for calculating the size of objects imaged within the chamber, and also to orient the images or video such that the lines extend horizontally across the image when it is viewed on a graphical display.

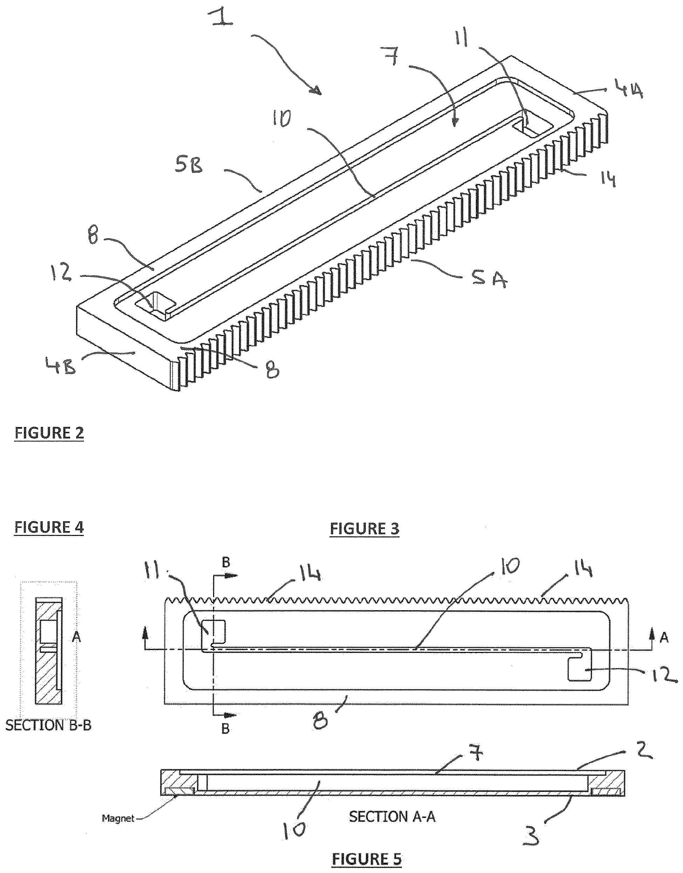

The side 5A of the slide 1 has a series of teeth 14 running the full length of the side. The purpose of the teeth 14 is to engage a drive mechanism of the slide reader 30 as is described in more detail below. Referring to Figures 3 to 5, the inlet chamber 11 and outlet chamber 12 are about the same depth as the sample chamber 16, and have a width and length of about 5 mm. This has been found to be sufficiently large to allow it fill with sample liquid without the generation of bubbles.

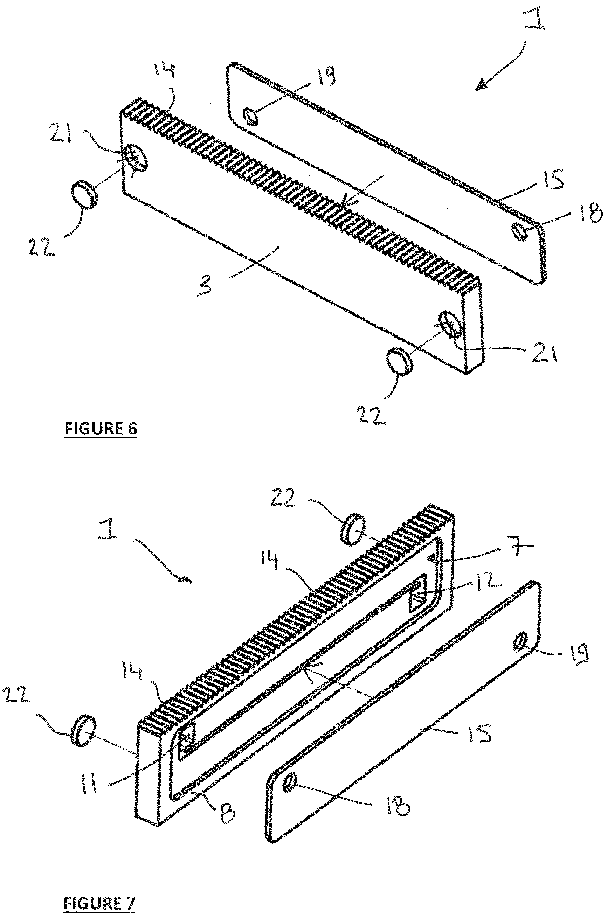

Referring to Figures 6 and 7, which show the slide in exploded views, the lower surface 3 of the slide 1 has detents 21 disposed at each end of the slide and dimensioned to receive disk-shaped magnets 22. In use, and as described in more detail below, the magnets help keep the slide stable when it is being moved inside the slide reader 30.

Referring to Figures 8 to 17, the slide reader 30 and attachment module 40 are described. The slide reader comprises a housing 31 made up of a lens module 32, a slide receiving module 33 and a base module 34. The lens module 32 has an upper surface having an aperture 33 aligned with a downwardly projecting lens 34. In this embodiment, the lens comprises a 10x objective lens and a 20x eyepiece lens. The base module 34 comprises a housing 35 and contains an electric motor 36 and associated circuitry. The slide reader 30 and attachment module 40 are shown in an exploded form in Figure 9.

The attachment module 40 has a square planar shape with an upper surface 41, lower surface 42, and a central aperture 43 that extends through the module providing a viewing hole for the imaging device (e.g. a camera of a mobile phone). The upper surface 41 has a circular recess and a circular film of synthetic setae 44 is nested in the recess. The synthetic setae provides a dry adhesive for securely fixing a mobile phone to the upper surface with the camera aligned with the central aperture 43. As the attachment module 40 is detachable from the slide reader 30 and can be easily held and articulated in one hand, the task of aligning the camera of a phone with the module such that the camera is aligned with the central aperture is easily performed. The lower surface 42 of the attachment module is configured to detachable attach to the slide reader 30 in a correct alignment with the central aperture 43 aligned perfectly with the aperture 33 of the slide reader. In this embodiment, the lower surface of the attachment module includes magnets 44 disposed on each side of the central aperture 43, and corresponding magnets 44 are mounted on the upper surface of the slide reader on each side of the aperture 33. In addition, a periphery of the lower surface 41 of the attachment module 40 and upper surface of the slide reader comprises corresponding chamfered sections 46 configured to assist with aligning the attachment module and slide reader.

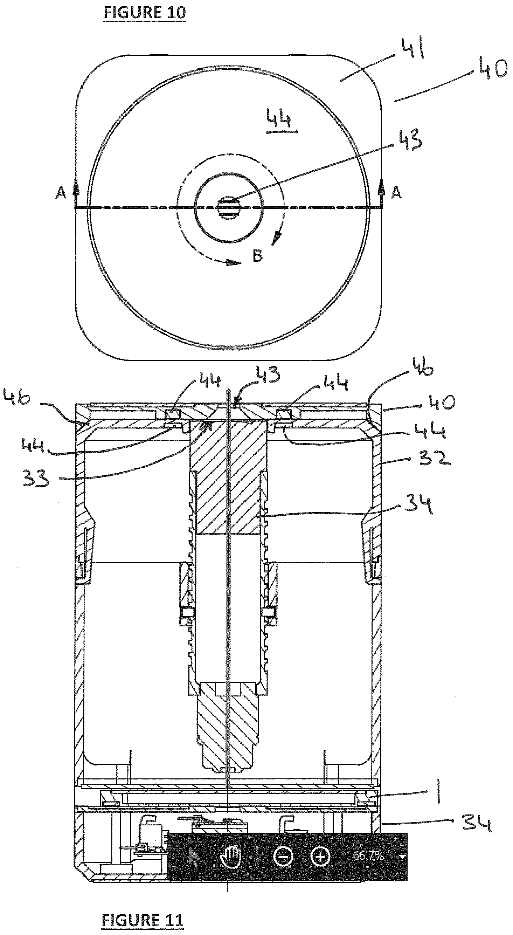

Figure 10 is a detailed top plan view of the kit looking through the central aperture 43 of the attachment module showing the view of the slide through the lens. Figure 11 is a sectional view through the kit showing the alignment of the apertures 43, 33 in the attachment module and slide reader and the lens 34.

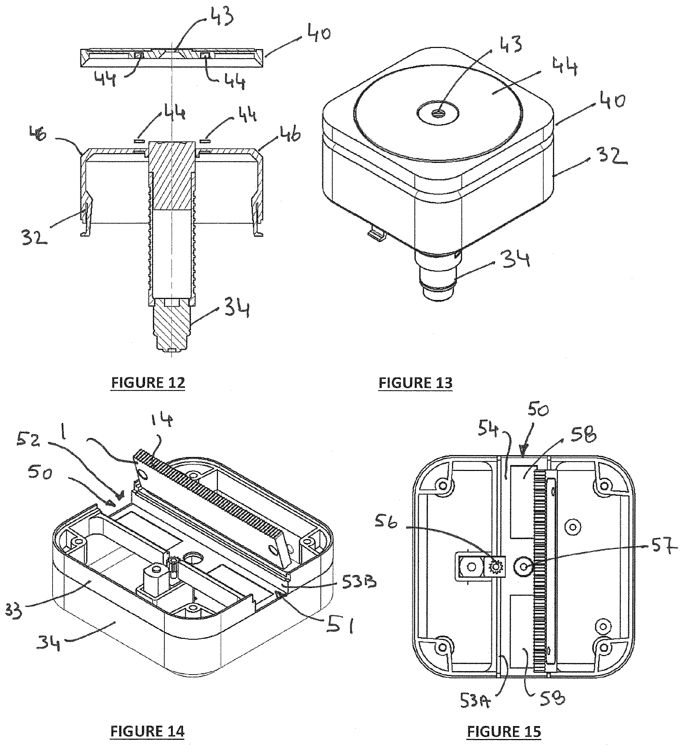

Figures 14 and 15 illustrated the lower part of the slide reader including the slide receiving module 33 and base module 34. The slide receiving module 33 includes a central slide receiving track 50 with a slide receiving end 51, slide dispensing end 52, sidewalls 53A and 53B and a base 54. The dimensions of the track are configured to receive the slide and allow unhindered movement of the slide along the track in a linear motion. One of the sidewalls 53A has a gap that contains a pinion wheel 56 configured to engage the teeth 14 on a side of the slide. The pinion wheel is mounted for rotation about an axis that is perpendicular to a longitudinal axis of the track, and is coupled to the electric motor 36. As illustrated in Figure 14, the base 54 of the track 50 comprises a central aperture 57 that is aligned with the lens 34 and apertures 33 and 34, and magnetic strips 58 disposed on each side of the aperture 57. The purpose of the magnetic strips is to attract the magnets on the lower surface of the slide to provide downforce to the slide during its movement along the track, thereby preventing any vertical movement of the slide relative to the slide reader.

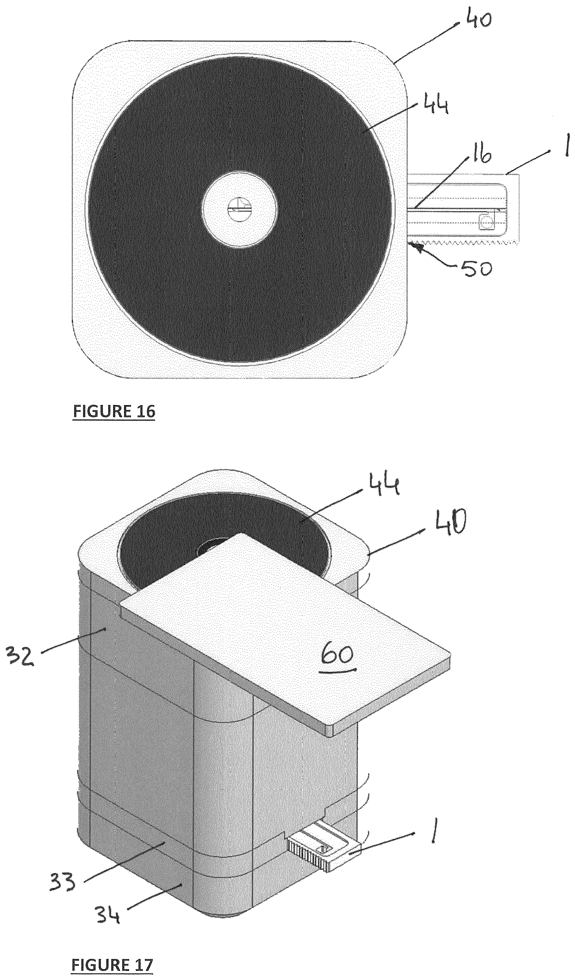

Figure 16 is a top plan view of the kit of Figure 8 showing the phone attachment module and a slide partly inserted into the slide receiving module with an inlet end of the liquid sample chamber and first indicium visible through the apertures.

Figure 17 illustrates a mobile phone attached to the attachment module with the phone camera aligned with the central aperture of the attachment module.

In use, a liquid sample is prepared. In the case of faecal egg analysis, this generally involves mixing a sample of faeces from an animal with a flotation fluid and filtering the mixture to provide the liquid sample. The sample is then injected through an inlet aperture of the slide via the inlet chamber which serves to inhibit bubble formation. Generally about 2 to 4 ml of sample is injected into the slide. A mobile phone is then attached the upper surface of the attachment module with the camera of the phone aligned with the central aperture of the attachment module. Alignment can be easily checked during and after alignment by viewing the central aperture from the lower surface of the module. The attachment module is then placed on top of the slide reader where it slots into place, aided by the corresponding chamfering and magnets on the abutting surfaces.

The slide is them gently inserted into the track until the teeth on the slide engage the pinion wheel. At this point of insertion (shown in Figure 19), the first indicium and inlet of the liquid sample chamber will be visible through the aperture. A button is then pressed to start the motor and move the slide along the track at a speed of 3.14 mm/s. At this initial stage of imaging, the camera/phone is trained to recognise the first indicium and auto-focus the lens of the camera on the first indicium, which serves to focus the camera on the same plane as the top of the liquid sample chamber. Recognition of the first indicium also prompts the camera/phone to start recording video of the full width of the sample chamber as it moves along the track and across the field of view. Recognition of the second indicium prompts the camera/phone to stop recording. The phone is also configured to detect if the second indicium is in focus, and if it is not in focus it will send a message to the user to indicate that the recorded video of the length of the sample chamber may not be usable. The lines on each side of the chamber may be used by the phone/device as a dimensional reference to calculate the size of microscopic objects in the field of view, for example faecal eggs. The data recorded by the phone is sent to a remote server using the communications functionality of the phone and analysed and analysis data is sent back to the phone. The analysis data may be an
indication of the health of an animal, for example whether or not it has a parasitic infection, the type or severity of the infection, whether it should be treated with an anti-parasite medication, or the type of medication to be employed. The functionality of the phone when interacting with the images of the slide is generally controlled by downloadable software which forms part of the kit of the invention. The software may be updated on the mobile device/phone via a wireless or wired connection.

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A mobile kit for imaging microscopic organisms in a liquid sample, comprising:
a slide (1) having a linear elongated liquid sample chamber (16) with a liquid sample inlet; and
a slide reader (30) containing a lens (34) and configured to receive the slide and image the liquid sample chamber (16) through the lens (34) using a mobile imaging device (60) that is coupled to the slide reader (30), the slide reader (30) further comprising:
an upper surface comprising an aperture (33) aligned with the lens (34);
a track (50) configured to receive the slide (1) and guide movement of the slide (1) along the track allowing the length of the linear elongated liquid sample chamber (16) to be imaged through the lens (34); and
a drive module configured to move the slide (1) along the track (50); and
a substantially planar attachment module (40) having an upper surface (41), a lower surface (42), and an aperture (43) extending between the surfaces, wherein the upper surface (41) is configured for detachable attachment to a mobile imaging device (60) and the lower surface (42) is configured to detachably attach to the upper surface of the slide reader (30) with the aperture (43) in the lower surface of the attachment module (40) aligned with the aperture (33) in the upper surface of the slide reader (30), such that a lens of the mobile imaging device (60) is aligned with the lens (34) of the slide reader (30) through the aligned apertures (33, 43).

2. A mobile kit according to claim 1, in which the lower surface (42) of the attachment module (40) and the upper surface of the slide reader (30) are configured to correctly align the attachment module (40) and slide reader (30) such that the mobile imaging device (60) is aligned with the lens (34) of the slide reader (30) through the aperture (43).

3. A mobile kit according to claim 2 in which the upper surface of the slide reader (30) and the lower surface (42) of the attachment module (40) are shaped or comprise corresponding magnets (44) to guide the alignment of the attachment module (40) and slide reader (30).

4. A mobile kit according to any of claims 2 to 4, in which the upper surface of the attachment module comprises a layer of synthetic setae (44).

5. A mobile kit according to any preceding claim, in which the slide reader comprises a lens module (32), a base module, and a slide receiving module containing the track and drive mechanism connected between the lens module and base module.

6. A mobile kit according to claim 5, in which the lens module (32) is detachably attached to the slide receiving module.

7. A mobile kit according to any preceding claim, in which the track (50) comprises an illumination section disposed directly under the lens (34), and the slide reader (30) comprises a light disposed under the illumination section to direct light through the illumination section on to a base of the slide during use.

8. A mobile kit according to any preceding claim, in which the track (50) is disposed horizontally across the slide reader and is configured to receive the slide on one side of the slide reader housing and dispense the slide on an opposite side of the slide reader housing.

9. A mobile kit according to any preceding claim, in which the lens (34) of the slide reader (30) is configured to provide a field of view when imaged by the mobile imaging device, in which the width of the linear elongated liquid sample chamber comprises at least 60% of the field of view.

10. A mobile kit according to any preceding claim, in which the slide (1) has an upper face and a first indicium (70) disposed on the upper face at an inlet end of the slide in a position that is axially aligned with a longitudinal axis of the linear elongated liquid sample chamber.

11. A mobile kit according to claim 10, including a second indicium (71) disposed on the upper face at an outlet end of the slide in a position that is axially aligned with a longitudinal axis of the linear elongated liquid sample chamber.

12. A mobile kit according to any preceding claim, in which the upper face of the slide comprises a visible line disposed on each longitudinal side of the linear elongated liquid sample chamber and co-parallel and optionally co-extensive with the linear elongated liquid sample chamber.

13. A mobile kit according to claim 10, comprising software for a mobile communications device having a camera, in which the software is configured to:
cause the mobile communications device to: recognise using the camera the first indicium (70) disposed on the upper face of the slide (1) adjacent an inlet end of the linear elongated liquid sample chamber; and focus the lens of the camera on the first indicium; or
cause the mobile communications device to: recognise using the camera the second indicium (71) disposed on the upper face of the slide (1) adjacent an outlet end of the linear elongated liquid sample chamber; determine if the second indicium is in focus; and alert a user if the second indicium is not in focus; or
recognise using the camera the first indicium (70) disposed on the upper face of the slide adjacent an inlet end of the linear elongated liquid sample chamber; initiate recording of images or video by the camera along the length of the linear elongated liquid sample chamber in response to recognition of the first indicium; recognise using the camera the second indicium (71) disposed on the upper face of the slide (1) adjacent an outlet end of the linear elongated liquid sample chamber; and stop recording of images or video by the camera in response to recognition of the second indicium.

14. A method of imaging microscopic organisms in a liquid sample that employs a mobile kit of any of claims 1 to 13, the method comprising the steps of:
loading a liquid sample into the linear elongated liquid sample chamber (16) of the slide (1);
placing the slide (1) into the track (50) of the slide reader (30);
attaching a mobile imaging device (60) to the attachment module (40) such that the mobile imaging device can image through the aperture (43) of the attachment module (40);
attaching the attachment module (40) to the slide reader (30) with the aperture (43) in the lower surface of the attachment module (40) aligned with the aperture (33) in the upper surface of the slide reader (30), such that a lens of the mobile imaging device (60) is aligned with the lens (34) of the slide reader (30) through the aperture (43);
actuating the drive mechanism to move the slide (1) along the track (50) in a linear motion such that the field of view of the lens (34) moves along the length of the linear elongated liquid sample chamber (16) and
recording images or a video of the length of the linear elongated liquid sample chamber (16) by the mobile imaging device (60).

15. A method according to claim 14, including a step of recording images or a video of the full length of the linear elongated liquid sample chamber by the mobile imaging device (60).

## Patentansprüche

1. Ein mobiles Kit zum Abbilden von mikroskopischen Organismen in einer flüssigen Probe, das Folgendes aufweist:
einen Objektträger (1) mit einer linearen, länglichen Kammer für eine flüssige Probe bzw. Flüssigkeitsprobenkammer (16) mit einem Flüssigkeitsprobeneinlass; und
einen Objektträgerleser (30), der eine Linse (34) enthält, und der so konfiguriert ist, dass er den Objektträger aufnimmt und die Flüssigkeitsprobenkammer (16) durch die Linse (34) mithilfe eines mobilen Bildgebungsgeräts (60) abbildet, das mit dem Objektträgerleser (30) gekoppelt ist, wobei der Objektträgerleser (30) ferner Folgendes aufweist:
eine obere Fläche, die eine Öffnung (33) aufweist, die mit der Linse (34) ausgerichtet ist;
eine Schiene (50), die so konfiguriert ist, dass sie den Objektträger (1) aufnimmt und eine Bewegung des Objektträgers (1) entlang der Schiene führt, sodass die Länge der linearen, länglichen Flüssigkeitsprobenkammer (16) durch die Linse (34) abgebildet werden kann; und
ein Antriebsmodul, das so konfiguriert ist, dass es den Objektträger (1) entlang der Schiene (50) bewegt; und
ein im Wesentlichen planares Befestigungsmodul (40) mit einer oberen Fläche (41), einer unteren Fläche (42), und mit einer sich zwischen den Flächen erstreckenden Öffnung (43), wobei die obere Fläche (41) zur abnehmbaren Befestigung an einem mobilen Bildgebungsgerät (60) und die untere Fläche (42) zur abnehmbaren Befestigung an der oberen Fläche des Objektträgerlesers (30) mit der Öffnung (43) in der unteren Fläche des Befestigungsmoduls (40) ausgerichtet ist, das mit der Öffnung (33) in der oberen Fläche des Objektträgerlesers (30) ausgerichtet ist, sodass eine Linse des mobilen Bildgebungsgeräts (60) durch die ausgerichteten Öffnungen (33, 43) mit der Linse (34) des Objektträgerlesers (30) ausgerichtet ist.

2. Ein mobiles Kit gemäß Anspruch 1, bei dem die untere Fläche (42) des Befestigungsmoduls (40) und die obere Fläche des Objektträgerlesers (30) so konfiguriert sind, dass das Befestigungsmodul (40) und der Objektträgerleser (30) korrekt ausgerichtet sind, sodass das mobile Bildgebungsgerät (60) durch die Öffnung (43) hindurch mit der Linse (34) des Objektträgerlesers (30) ausgerichtet ist.

3. Ein mobiles Kit gemäß Anspruch 2, wobei die obere Fläche des Objektträgerlesers (30) und die untere Fläche (42) des Befestigungsmoduls (40) entsprechend geformt sind oder entsprechende Magnete (44) aufweisen, um die Ausrichtung des Befestigungsmoduls (40) und des Objektträgerlesers (30) zu führen.

4. Ein mobiles Kit gemäß einem der Ansprüche 2 bis 4, wobei die obere Fläche des Befestigungsmoduls eine Schicht aus synthetischen Borsten (44) aufweist.

5. Ein mobiles Kit gemäß einem der vorhergehenden Ansprüche, bei dem der Objektträgerleser ein Linsenmodul (32), ein Basismodul und ein Objektträger-Aufnahmemodul aufweist, das die Schiene und den Antriebsmechanismus enthält, die zwischen dem Linsenmodul und dem Basismodul verbunden sind.

6. Ein mobiles Kit gemäß Anspruch 5, bei dem das Linsenmodul (32) abnehmbar am Objektträger-Aufnahmemodul befestigt ist.

7. Ein mobiles Kit gemäß einem der vorhergehenden Ansprüche, bei dem die Schiene (50) einen direkt unter der Linse (34) angeordneten Beleuchtungsabschnitt aufweist und wobei der Objektträgerleser (30) eine unter dem Beleuchtungsabschnitt angeordnete Lichtquelle aufweist, um während des Gebrauchs Licht durch den Beleuchtungsabschnitt auf eine Basis des Objektträgers zu lenken.

8. Ein mobiles Kit gemäß einem der vorhergehenden Ansprüche, bei dem die Schiene (50) horizontal über dem Objektträgerleser angeordnet und so konfiguriert ist, dass sie den Objektträger auf einer Seite des Gehäuses des Objektträgerlesers aufnimmt und den Objektträger auf einer gegenüberliegenden Seite des Gehäuses des Objektträgerlesers ausgibt.

9. Ein mobiles Kit gemäß einem der vorhergehenden Ansprüche, bei dem die Linse (34) des Objektträgerlesers (30) so konfiguriert ist, dass sie ein Sichtfeld bereitstellt, wenn sie von dem mobilen Bildgebungsgerät abgebildet wird, wobei die Breite der linearen, länglichen Flüssigkeitsprobenkammer mindestens 60 % des Sichtfeldes aufweist.

10. Ein mobiles Kit gemäß einem der vorhergehenden Ansprüche, bei dem der Objektträger (1) eine obere Fläche und eine erste Kennzeichnung (70) aufweist, die auf der oberen Fläche an einem Einlassende des Objektträgers in einer Position angeordnet ist, die axial mit einer Längsachse der linearen, länglichen Flüssigkeitsprobenkammer ausgerichtet ist.

11. Ein mobiles Kit gemäß Anspruch 10, das eine zweite Kennzeichnung (71) aufweist, die auf der oberen Fläche an einem Auslassende des Objektträgers in einer Position angeordnet ist, die axial mit einer Längsachse der linearen, länglichen Flüssigkeitsprobenkammer ausgerichtet ist.

12. Ein mobiles Kit gemäß einem der vorhergehenden Ansprüche, bei dem die obere Fläche des Objektträgers eine sichtbare Linie aufweist, die an jeder Längsseite einer länglichen Flotationskammer angeordnet ist und die parallel und optional gleich lang wie die lineare, längliche Flüssigkeitsprobenkammer ist.

13. Ein mobiles Kit gemäß Anspruch 10, das Software für ein mobiles Kommunikationsgerät mit einer Kamera aufweist, in dem die Software so konfiguriert ist, dass sie:
das mobile Kommunikationsgerät dazu veranlasst: unter Verwendung der Kamera die erste Kennzeichnung (70) zu erkennen, die auf der oberen Fläche des Objektträgers (1) neben einem Einlassende der linearen, länglichen Flüssigkeitsprobenkammer angeordnet ist, und die Linse der Kamera auf die erste Kennzeichnung zu fokussieren, oder
das mobile Kommunikationsgerät dazu veranlasst, unter Verwendung der Kamera, die zweite Kennzeichnung (71) zu erkennen, die auf der oberen Fläche des Objektträgers (1) neben einem Auslassende der linearen, länglichen Probenkammer angeordnet ist; bestimmt, ob die zweite Kennzeichnung scharfgestellt ist; und einen Benutzer warnt, falls die zweite Kennzeichnung nicht scharfgestellt ist; oder
unter Verwendung der Kamera die erste Kennzeichnung (70) erkennt, die auf der oberen Fläche des Objektträgers neben einem Einlassende der linearen, länglichen Probenkammer angeordnet ist; eine Aufzeichnung von Bildern oder Videos durch die Kamera entlang der Länge der linearen, länglichen Probenkammer initiiert als Reaktion auf eine Erkennung der ersten Kennzeichnung; unter Verwendung der Kamera die zweite Kennzeichnung (71) erkennt, die auf der oberen Fläche des Objektträgers (1) neben einem Auslassende der linearen, länglichen Probenkammer angeordnet ist; und die Aufzeichnung von Bildern oder Videos durch die Kamera als Reaktion auf die Erkennung der zweiten Kennzeichnung beendet.

14. Ein Verfahren zum Abbilden mikroskopischer Organismen in einer Flüssigkeitsprobe, das ein mobiles Kit gemäß einem der Ansprüche 1 bis 13 verwendet, wobei das Verfahren die folgenden Schritte aufweist:
Einfüllen einer Flüssigkeitsprobe in die lineare, längliche Flüssigkeitsprobenkammer (16) des Objektträgers (1);
Platzieren des Objektträgers (1) in der Schiene (50) des Objektträgerlesers (30);
Befestigen eines mobilen Bildgebungsgeräts (60) an dem Befestigungsmodul (40), so dass das mobile Bildgebungsgerät durch die Öffnung (43) des Befestigungsmoduls (40) Bilder aufnehmen kann;
Befestigen des Befestigungsmoduls (40) an dem Objektträgerleser (30), wobei die Öffnung (43) in der unteren Fläche des Befestigungsmoduls (40) mit der Öffnung (33) in der oberen Fläche des Objektträgerlesers (30) ausgerichtet ist, so dass eine Linse des mobilen Bildgebungsgeräts (60) durch die Öffnung (43) auf die Linse (34) des Objektträgerlesers (30) ausgerichtet ist;
Betätigen des Antriebsmechanismus, um den Objektträger (1) entlang der Schiene (50) in einer linearen Bewegung zu bewegen, so dass sich das Sichtfeld der Linse (34) entlang der Länge der linearen, länglichen Flüssigkeitsprobenkammer (16) bewegt, und
Aufzeichnen von Bildern oder eines Videos der Länge der linearen, länglichen Flüssigkeitsprobenkammer (16) durch das mobile Bildgebungsgerät (60).

15. Ein Verfahren gemäß Anspruch 14, einschließlich eines Schrittes zum Aufzeichnen von Bildern oder eines Videos der gesamten Länge der linearen, länglichen Flüssigkeitsprobenkammer durch das mobile Bildgebungsgerät (60).

## Revendications

1. Kit mobile d'imagerie de micro-organismes dans un échantillon liquide, comprenant :
une lame (1) ayant une chambre d'échantillon liquide allongée linéaire (16) avec une admission d'échantillon liquide ; et
un lecteur de lames (30) contenant une lentille (34) et configuré pour recevoir la lame et pour imager la chambre d'échantillon liquide (16) au moyen de la lentille (34) en utilisant un dispositif d'imagerie mobile (60) qui est relié au lecteur de lames (30), le lecteur de lames (30) comprenant en outre :
une surface supérieure comprenant une ouverture (33) alignée avec la lentille (34) ;
un rail (50) configuré pour recevoir la lame (1) et guider le mouvement de la lame (1) le long du rail ce qui permet d'imager la longueur de la chambre d'échantillon liquide allongée linéaire (16) au moyen de la lentille (34) ; et
un module d'entraînement configuré pour déplacer la lame (1) le long du rail (50) ; et
un module de fixation sensiblement plan (40) ayant une surface supérieure (41), une surface inférieure (42) et une ouverture (43) s'étendant entre les surfaces, dans lequel la surface supérieure (41) est configurée pour se fixer de façon amovible à un dispositif d'imagerie mobile (60) et la surface inférieure (42) est configurée pour se fixer de façon amovible à la surface supérieure du lecteur de lames (30) avec l'ouverture (43) dans la surface inférieure du module de fixation (40) qui est alignée avec l'ouverture (33) dans la surface supérieure du lecteur de lames (30), de sorte qu'une lentille du dispositif d'imagerie mobile (60) soit alignée avec la lentille (34) du lecteur de lames (30) à travers les ouvertures alignées (33, 43).

2. Kit mobile selon la revendication 1, dans lequel la surface inférieure (42) du module de fixation (40) et la surface supérieure du lecteur de lames (30) sont configurées pour aligner correctement le module de fixation (40) et le lecteur de lames (30) de sorte que le dispositif d'imagerie mobile (60) soit aligné avec la lentille (34) du lecteur de lames (30) à travers l'ouverture (43).

3. Kit mobile selon la revendication 2, dans lequel la surface supérieure du lecteur de lames (30) et la surface inférieure (42) du module de fixation (40) sont façonnées ou comprennent des aimants correspondants (44) pour guider l'alignement du module de fixation (40) et du lecteur de lames (30).

4. Kit mobile selon l'une quelconque des revendications 2 à 4, dans lequel la surface supérieure du module de fixation comprend une couche de soies synthétiques (44).

5. Kit mobile selon l'une quelconque des revendications précédentes, dans lequel le lecteur de lames comprend un module de lentille (32), un module de base et un module de réception de lames contenant le mécanisme de rail et d'entraînement connecté entre le module de lentille et le module de base.

6. Kit mobile selon la revendication 5, dans lequel le module de lentille (32) est fixé de façon amovible au module de réception de lames.

7. Kit mobile selon l'une quelconque des revendications précédentes, dans lequel le rail (50) comprend une section d'éclairage disposée directement sous la lentille (34), et le lecteur de lames (30) comprend une lumière disposée sous la section d'éclairage pour diriger la lumière à travers la section d'éclairage sur une base de la lame pendant son utilisation.

8. Kit mobile selon l'une quelconque des revendications précédentes, dans lequel le rail (50) est disposé horizontalement en travers du lecteur de lames et est configuré pour recevoir la lame d'un côté du boîtier du lecteur de lames et distribuer la lame d'un côté opposé du boîtier du lecteur de lames.

9. Kit mobile selon l'une quelconque des revendications précédentes, dans lequel la lentille (34) du lecteur de lames (30) est configuré pour fournir un champ de vision lorsqu'il est imagé par le dispositif d'imagerie mobile, dans lequel la largeur de la chambre d'échantillon liquide allongée linéaire comprend au moins 60 % du champ de vision.

10. Kit mobile selon l'une quelconque des revendications précédentes, dans lequel la lame (1) a une face supérieure et un premier repère (70) disposé sur la face supérieure à une extrémité d'admission de la lame dans une position qui est alignée axialement avec un axe longitudinal de la chambre d'échantillon liquide allongée linéaire.

11. Kit mobile selon la revendication 10, comportant un deuxième repère (71) disposé sur la face supérieure à une extrémité de sortie de la lame dans une position qui est alignée axialement avec un axe longitudinal de la chambre d'échantillon liquide allongée linéaire.

12. Kit mobile selon l'une quelconque des revendications précédentes, dans lequel la face supérieure de la lame comprend une ligne visible disposée de chaque côté longitudinal de la chambre de flottation allongée et co-parallèle et éventuellement co-extensive avec la chambre d'échantillon liquide allongée linéaire.

13. Kit mobile selon la revendication 10, comprenant un logiciel pour un dispositif de communication mobile ayant une caméra, dans lequel le logiciel est configuré pour :
amener le dispositif de communication mobile à : reconnaître en utilisant la caméra le premier repère (70) disposé sur la face supérieure de la lame (1) à proximité d'une extrémité d'admission de la chambre d'échantillon de liquide allongée linéaire ; et focaliser la lentille de la caméra sur le premier repère ; ou
amener le dispositif de communication mobile à : reconnaître en utilisant la caméra le deuxième repère (71) disposé sur la face supérieure de la lame (1) à proximité d'une extrémité de sortie de la chambre d'échantillon allongée linéaire ; déterminer si le deuxième repère est net ; et alerter un utilisateur si le deuxième repère n'est pas net ; ou
reconnaître en utilisant la caméra le premier repère (70) disposé sur la face supérieure de la lame à proximité d'une extrémité d'admission de la chambre d'échantillon allongée linéaire ; déclencher l'enregistrement d'images ou de vidéos par la caméra sur la longueur de la chambre d'échantillon allongée linéaire en réponse à la reconnaissance du premier repère ; reconnaître en utilisant la caméra le deuxième repère (71) disposé sur la face supérieure de la lame (1) à proximité d'une extrémité de sortie de la chambre d'échantillon allongée linéaire ; et arrêter l'enregistrement d'images ou de vidéos par la caméra en réponse à la reconnaissance du deuxième repère.

14. Procédé d'imagerie d'organismes microscopiques dans un échantillon liquide qui utilise un kit mobile selon l'une quelconque des revendications 1 à 13, le procédé comprenant les étapes suivantes :
le chargement d'un échantillon liquide dans la chambre d'échantillon liquide allongée linéaire (16) de la lame (1) ;
le placement de la lame (1) dans le rail (50) du lecteur de lames (30) ;
la fixation d'un dispositif d'imagerie mobile (60) au module de fixation (40) de sorte que le dispositif d'imagerie mobile puisse prendre des images à travers l'ouverture (43) du module de fixation (40) ;
la fixation du module de fixation (40) au lecteur de lames (30) l'ouverture (43) dans la surface inférieure du module de fixation (40) étant alignée avec l'ouverture (33) dans la surface supérieure du lecteur de lames (30), de sorte qu'une lentille du dispositif d'imagerie mobile (60) soit alignée avec la lentille (34) du lecteur de lames (30) à travers l'ouverture (43) ;
l'actionnement du mécanisme d'entraînement pour déplacer la lame (1) le long du rail (50) de façon linéaire, de sorte que le champ de vision de la lentille (34) se déplace le long de la longueur de la chambre d'échantillon liquide allongée linéaire (16) et
l'enregistrement d'images ou d'une vidéo de la longueur de la chambre d'échantillon allongée linéaire (16) par le dispositif d'imagerie mobile (60).

15. Procédé selon la revendication 14, comportant une étape d'enregistrement d'images ou d'une vidéo de toute la longueur de la chambre d'échantillon liquide allongée linéaire par le dispositif d'imagerie mobile (60).
